(19) Europäisches Patentamt
European Patent Office
Office européen des brevets

(11) Publication number: **0 246 861 B1**

(12) **EUROPEAN PATENT SPECIFICATION**

(45) Date of publication of patent specification: **17.03.93** (51) Int. Cl.$^5$: **A61K 35/58**

(21) Application number: **87304427.5**

(22) Date of filing: **19.05.87**

(54) **Use of compositions based on crotoxine, for the manufacture of a medicament for the treatment of carcinomas.**

(30) Priority: **19.05.86 AR 304006**
**02.06.86 AR 304195**

(43) Date of publication of application:
**25.11.87 Bulletin 87/48**

(45) Publication of the grant of the patent:
**17.03.93 Bulletin 93/11**

(84) Designated Contracting States:
**AT BE CH DE ES FR GB GR IT LI NL SE**

(56) References cited:
**EP-A- 0 063 091**
**DE-C- 675 605**

**CHEMICAL ABSTRACTS, vol. 83, no. 11, 15 September 1975, Columbus, OH (US); p. 19, no. 90676r A.T. Tu et al.**

**CHEMICAL ABSTRACTS, vol. 96, no. 16, 19 April 1982, Columbus, OH (US); p. 46, no. 135479r F.S. Markland et al.**

**CHEMICAL ABSTRACTS, vol. 94, no. 11, 16 March 1981, Columbus, OH (US); no. 77706h H. Fraenkel-Conrat et al.**

**CHEMICAL ABSTRACTS, vol. 98, no. 18, 02 May 1983, Columbus, OH (US); no. 155921y H. Fraenkel-Conrat et al.**

(73) Proprietor: **Ventech Research, Inc.**
**38 Sidney Street**
**Cambridge, Massachusetts 02139(US)**

(72) Inventor: **Vidal, Juan Carlos**
**5550 South Dorchester, Apt. 1106**
**Chicago, IL 60637(US)**
Inventor: **Costa, Luis Alberto**
**Las Heras 3898, 1 "C"**
**AR-1425 Buenos Aires(AR)**
Inventor: **Hernandez Plata, Guillermo Jose**
**Santa Fe 3435, 4"C"**
**AR-1425 Buenos Aires(AR)**
Inventor: **Coni Molina, Carlos Maria**
**Salta 1436, 3 "A"**
**AR-1137 Buenos Aires(AR)**

(74) Representative: **Pearce, Anthony Richmond et al**
**MARKS & CLERK Alpha Tower Suffolk Street**
**Oueensway Birmingham B1 1TT (GB)**

EP 0 246 861 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

Technical Field of the Invention

This invention relates to the use of a substantially pure crotoxin complex of crotoxin A and B sub-units for the manufacture of a therapteutic composition for the treatment of malignant tumours.

Description of Prior Art

The analgesic effect of snake venom has been known since antiquity and numerous authors have pointed out the efficiency of the administration of crude cobra and rattle-snake venoms in the treatment of trigeminal neuralgias, and tabetic and tumoral algias. In the case of tumoral algias, patients could be maintained without the administration of morphine and without pain in 70 per cent of the cases.

However, few authors followed up these patients. The appearance of new analgesics cast aside research in this field and the progress of chemotherapy led to the abandonment of research in the possible antineoplastic action of venoms. Obviously, at that time crude venoms were employed without an adequate classification. Sometimes, cobra venom from India or South Africa were employed indiscriminately with unpredictable results.

Independently, the first cytotoxic component to be isolated and purified to homogeneity from snake venom was a cytotoxin, obtained by BRAGANCA et al (1967) from Naja naja venom. Later on, TAKECHI et al (1971) isolated two cytotoxins from the same venom having high cytotoxic activity on tumor cells. GILLO (1966), WIRTHEINER & GILLO (1966), BRISBOIS et al (1968), proved that the Naja naja venom inhibits growth of tumours in animals (mice) as the tumor cells are incapable of proliferating, although said cells are not destroyed.

However, the complexity in the composition of venoms and the absence of adequate controls led researchers to believe that the cytotoxic action against tumour cells would not be selective when confronted with normal cells.

In contrast with cytotoxins from elapid venoms (ie, cobra venom), which are peptides of 59-62 strongly basic amino acids, crotalid venoms do not contain cytotoxins and their cytotoxic activity is related to other components.

The fraction identified as "crotoxin" from the venom of Crotalus durissus terrificus was isolated first by SLOTTA and FRAENKEL-CONRAT (1938) by thermal treatment of the venom and precipitation with alcohol. This fraction, which could be crystallized with pyridine-acetic acid to pH 4.7, and the studies on free electrophoresis (LI & FRAENKEL-CONRAT) and ultracentrifugation (GRALEN & SVEDBERG, 1938) did not illustrate gross unhomogeneities and allowed said fraction to be considered a homogenous chemical entity.

In 1971, two research groups proved that "crotoxin" is in fact a complex formed by two main components (RUBSAMEN et al, 1971; HENDON & FRAENKEL-CONRAT, 1971). One of said components is a phospholipase $A_2$ having a molecular weight of 14500 and isoelectric point 9.7, also named Crotoxin B (for basic). The other component is a peptide having a molecular weight of 9500 and isoelectric point of 3.5, and lacking enzymatic activity, named Crotoxin A (for acidic).

We have separated basic phospholipase $A_2$ (crotoxin B) and the acidic sub-unit (crotoxin A) from Crotalus durissus terrificus venom with a procedure consisting of:

(1) Chromatography on Sephadex G-75 at pH 4.5, and

(2) Ion Exchange Chromatography on CM-Sephadex C-50 and elution with ammonium formate having a concave molarity gradient of from 0.1 to 1.0 M (pH 3.5), followed by a linear gradient (1.0 to 3.0 M) of the same buffer. In this step the following components were separated:

1) Crotoxin A;

2) A phospholipase $A_2$ having an isoelectric point of 4.1;

3) A third component identifiable as Crotamine (contaminant), and

4) Basic phospholipase $A_2$ (Crotoxin B) which is last to be eluted.

Crotoxin A is further purified by chromatography on DEAE-Sephadex A-50 with potassium phosphate (pH 6.5) as eluent. Crotoxin B is also further purified by chromatography on CM-Sephadex C-50 (pH 3.5).

The purified fractions are concentrated by ultra-filtration on a Diaflo UM-10 membrane (AMICON CO., Danvers, Ma., U.S.A.) and equilibrated with 0.15 M NaCl by extensive dialysis. The purified components behave as homogeneous materials by electrophoresis in polyacrylamide gel in the presence of sodium dodecylsulphate, as well as by amino acids composition. In the interest of clarity, when referring to sub-units A and B, same correspond to: Crotoxin A and Crotoxin B (phospholipase $A_2$), respectively, separated or in the complex form.

Summary of the Invention

According to one aspect of this invention, there is provided the use of a substantially pure crotoxin complex of crotoxin sub-units A and B for the manufacture of a therapeutic composition for the treatment of malignant tumours.

According to another aspect of this invention, there is provided a process for manufacturing a therapeutic composition containing a crotoxin complex of crotoxin A and B sub-units for the treatment of malignant tumours, said process comprising the steps of separating by chromatography the crotoxin complex from crude Crotalus venom; concentrating reunited fractions formed by eluting the absorbed crotoxin complex; dispersing in aqueous medium an insoluble product precipitated in said concentrating step; fractionating said sub-units A and B by chromatography of said dispersion in an adsorption column and eluting units A and B with a buffer solution (eg of ammonium formate having a pH of about 3.5), and purifying separated eluted fractions containing sub-units A and B; and eventually mixing said sub-units to form the crotoxin complex.

Detailed Description of the Invention

The present invention is based on the discovery by the applicants of certain antitumor properties of crotoxin A and crotoxin B.

1. ENZYMATIC PROPERTIES

A) Enzymatic Properties of Crotoxin B

Crotoxin B is a phospholipase $A_2$ and catalyses the hydrolysis of the ester bond between the fatty acid and the hydroxy group in position 2 of the glycerol moiety of 1,2-diacyl (1-alkenyl-2-acyl or 1-alkyl-2-acyl)-sn-glycero-3-phosphoglycerides (phospholipids), in accordance with the following scheme:

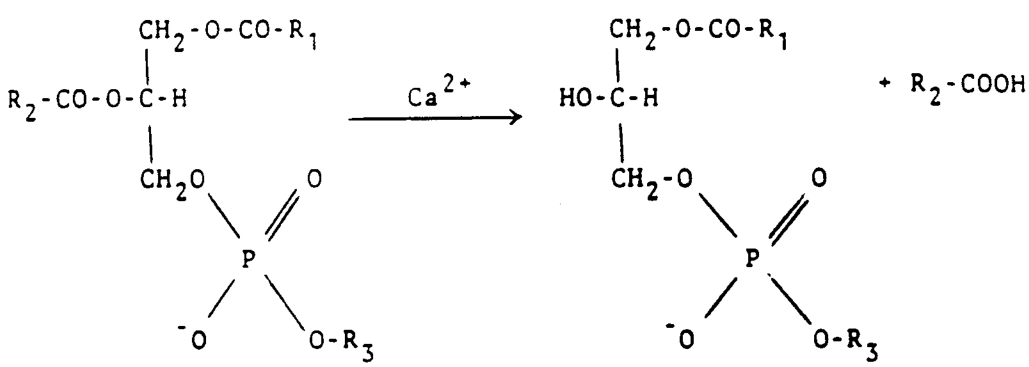


I (Phospholipids) II (Lysoderivative) III (fatty acid)

wherein $R_1$ and $R_2$ are fatty acid residues and $R_3$ can be: H (phosphatidic acid), a polyalcohol (glycerol in phosphatidylglycerol, inositol in phosphatidylinositol) or a nitrogenated alcohol (choline in phosphatidylcholine, ethanolamine in phosphatidylethanolamine, serine in phosphatidylserine). The reaction products are a free fatty acid (III) and the 1-acyl derivative (II) generally defined as lysoderivative (lysophosphatidinylcholine, lysophosphatidylethanolamine). The hydrolysis reaction is stereospecific, exhibiting position specificity by the ester bond of the C-2 of glycerol and requires specifically $Ca^{2+}$ ions as co-factor.

Crotoxin B hydroyzes phospholipids in different aggregation states, such as short chain lecithins obtained by chemical synthesis, and very soluble in water, aggregated as micelles as well as phospholipids with long-chain fatty acids ($C_{16}$-$C_{22}$) aggregated as vesicles or as liposomes, or in biologically important structures such as lipoproteins and biological membranes. Its specific activity on egg yolk lipoproteins (pH

8.0, 40 ° C) is of 700 umol of hydrolyzed substrate/min/mg of protein.

An important functional property in common with other lipolytic enzymes is that, in the presence of water soluble phospholipids, the substrate is bound to the active site and a productive ternary complex is formed in presence of $Ca^{2+}$. Then catalysis occurs and the hydrolysis products are released. When the phospholipid is aggregated (micelles or vesicles), the enzyme is adsorbed at the phospholipid-water interface with its active site oriented towards the ordered array of substrate molecules, it will bind a monomer molecule and, in the presence of $Ca^{2+}$ ions, an interfacial ternary complex will be formed. Hydrolysis will proceed by the same reaction mechanism but with a drastic increase in the rate of hydrolysis (10,000 to 100,000 fold).

This increase in the rate of hydrolysis resulting from the interaction of the enzyme with aggregated substrate is called "interfacial activation" and its mechanism has not been completely elucidated.

Crotoxin B is the crucial pharmacological agent of the crotoxin complex.

B) Crotoxin A: Its properties concerning the crotoxin complex.

Crotoxin A does not exhibit any detectable enzymatic or toxic activity, but it does exhibit two important properties concerning the mechanism of action of the complex, i.e.: a) when Crotoxin A is added to a solution of basic phospholipase $A_2$ (Crotoxin B) a highly stable complex is spontaneously formed ($Kd = 2x10^{-9} M$) having a lower isoelectric point (4.7) and a molecular weight similar to that of the native complex, and b) in the complex with Crotoxin A, the enzymatic activity of phospholipase $A_2$ is inhibited, mainly when the substrate is in aggregated state.

2. Pharmocodynamics - Mechanism of Action

The hydrolysis of phospholipids in the form of aggregates or biological membranes requires the binding of basic phospholipase $A_2$ to the phospholipid-water interface, and this binding occurs only after dissociation of the complex. We have used doubly labelled crotoxin complex ($I^{125}$ Crotoxin B and with $H^3$ Crotoxin A) demonstrating that crotoxin B only binds to membranes, while crotoxin A remains in the supernatant. We have found that the complex prepared with dimethylsuberimidate loses the ability to bind to membranes, although it still maintains phospholipase activity.

We have proved that the catalytic site of Crotoxin B in the crotoxin complex is accessible to the monomeric substrate and maintains its functional capacity intact.

However, isolated crotoxin B is able to firmly interact with phospholipid-water interfaces, while the complex with crotoxin A seems incapable of interacting with said interfaces.

The following conclusions can be arrived at from the above and other results:

(a) The formation of the complex between crotoxin B and crotoxin A does not affect the structure and properties of the active site of crotoxin B, but prevents binding of the enzyme to phospholipid-water interfaces and consequently inhibits enzymatic hydrolysis of substrates in the form of aggregates or biological membranes.

(b) The interaction of crotoxin B with aggregated substrates depends on the exposure of a specific area in the surface of the enzyme which is functionally and topographically different from the active site. Only the free enzyme, with that exposed surface, will be able to bind to interfaces and hydrolyze membrane phospholipids as well as to exhibit the phenomenon known as "interfacial activation".

(c) This specific region of the enzyme surface is the area occluded by the formation of the complex with crotoxin A, which implies that the interaction between crotoxin B with interfaces and crotoxin A are mutually exclusive, ie, the formation of the complex with crotoxin A prevents interaction of the enzyme with membranes and the interaction of crotoxin B with membranes prevents the formation of the complex with crotoxin A.

The observation of the cytopathic effect of the crotoxin complex on Ehrlich ascitic tumour cells was fortuitous, but it was verified that concentrations in the range of $10^{-9}$ M produced complete lysis of a culture starting at 60 minutes. However, its cytopathic action on hepatocytes, fibroblasts and peritoneal culture cells was poor.

In what concerns the mechanism of action, the following observations are important:-

(a) Crotoxin B is the only component of the crotoxin complex able to exert cytotoxic activity on tumor and normal cells. In both cases, the addition of isolated crotoxin B produced evidences of cellular damage.

(b) Cytotoxic action is related to the enzymatic activity of crotoxin B. Selective blocking of the active site by treatment with p-bromophenacyl bromide (CANZIANI et al., 1982) abolishes cytotoxic activity. The

discrete alterations which were observed appear to be related to the binding of the enzyme to the membrane, but these alterations apparently are not sufficient to cause lysis of the cells.

(c) The crotoxin complex presents however a more selective cytotoxic action on tumor cells.

(d) The applicant's investigations have proved that the crotoxin complex is able to bind to tumor cell membranes, so that the limiting step for the action of the crotoxin complex is the dissociation of sub-units A and B. A plausible explanation for this differential action of the crotoxin complex must reside in the existence of local physicochemical conditions at the level of tumor cell membranes promoting the dissociation of the complex. Said conditions prevailing in tumor cells may not be sufficient to cause significant dissociation of the crotoxin complex in the proximity of normal cells.

(e) Changes in plasmatic membranes of tumor cells have been known since 1958 and there is sufficient evidence to suggest that oncogenic agents alter numerous functions of plasmatic membranes consequently creating pleiomorphic changes observed in the membranes. In 1967, STOCKER described alterations in adhesiveness and the absence of contact inhibition, although these so not seem to be invariable properties in malignant tumors (WALLACH, 1973). Similar phenomena occur in electric uncoupling (WALLACH, 1973). The growth inhibition by contact, less prominent in tumor cells (STOCKER, 1967) and the greater fusogenic capacity (OKADA, 1969; POSTE, 1970) seem to run parallel with malignancy. Changes in surface potential of neoplastic cells have been observed (WALLACH, 1973) as well as permeability (SYLVEN et al, 1962) and immunological changes. The appearance of new antigens, embryonic antigens and deletion of certain selective antigens in organs have been observed in numerous experimental and spontaneous tumours. Dissociation of the crotoxin complex can be promoted by cooperative titration of the crotoxin A carboxylates. Although pH values of below 3 are necessary to produce dissociation, it must be borne in mind that these results have been obtained at equilibrium. The prevalent condition in a cell is the steady state, and therefore the degree of dissociation calculated at equilibrium may be considerably modified, particularly when gradients occur in restricted volumes within diffusion barriers.

(f) The net result of dissociation of the crotoxin complex in the proximity of tumoral cell plasmatic membranes is that crotoxin B binds to the membrane, producing hydrolysis of the membrane phospholipids. The hydrolysis products by phospholipase $A_2$ and particularly lysophosphatides (structure II above) have detergent properties (in this regard, the denomination of lysoderivatives is due to lytic properties). Thermodynamic and geometric reasons prevent said products from being packed in a stable lamellar structure. In model systems (liposomes) the addition of lysoderivates (1-3 mmol per 100 mmol of phospholipid) determines gross disturbances in permeability (for example, the release of sucrose and encapsulated cations, usually impermeable) and greater concentrations of lysoderivatives determine the disruption of the laminar structure and the formation of a mixed micellar aggregate.

Bearing in mind that membrane phospholipids contain long chain fatty acids (16 to 24 carbons), the hydrolysis products (Structures II and III above) do not necessarily abandon the membrane and the increase in their relative concentration brings about alterations causing the release of cytoplasmic marker enzymes into the medium and morphological alterations (swelling and disruption of mitochondrial and endoplasmic reticulum) leading to cellular lysis.

At present, it is not clear whether said alterations result from internalization of the enzyme or the abrupt changes in composition and ionic strength arising from permeability alterations.

## Conclusions

The mechanism producing tumor cell lysis can be explained as the result of enzymatic activity of crotoxin B. However, a point of great interest lies in the target selecting mechanism of the crotoxin complex. It is considered that the crotoxin complex acts as a circulating deposit of inert crotoxin B, exhibiting its activity only on membranes having physicochemical conditions which promote the dissociation of the crotoxin complex. Thus, these cells become vulnerable to attack by crotoxin B. Under these conditions, the tumour cell would be a more efficient alternative target for binding of crotoxin B.

In addition, it may be important to point out that phosphatidylinositol is among the phospholipids susceptible to being attacked by the enzyme. It is know at present that one of the critical signals inducing cellular proliferation is hydrolysis of triphosphoinositides by a membrane phospholipase C. Hydrolysis products are inositol-tri-phosphate which can act as second messenger and a di-glyceride responsible for the activation of protein quinase C which catalyzes phosphorylation of proteins. The hydrolysis product of phospholipase $A_2$, when acting on phosphatidylinositol is the corresponding lysoderivative, not an adequate substrate for endogenous phospholipase C, and therefore could interfere the cellular proliferation mechanism.

In accordance with the conventional protocol of the National Cancer Institute (NCI - USA), murine tumours melanoma B16, colon tumour 26, Ridgway's ostogenic sarcoma and Lewis carcinoma were used to evaluate antitumoral activity. In all cases, an increase in the average lifespan time was required exceeding 20% and/or inhibition of local growth greater than 50% to demonstrate activity in comparison with non-treated controls. Said requirement is slightly lower than that usually required (average lifespan exceeding 25%, growth inhibition exceeding 60%) due to the peculiar sensitivity of mice to the action of this toxin, as will be appreciated hereinafter.

The increase in the lifespan average time was between 150% and 300% with 55-80% of survivors after 90 days with melanoma B16; about 200% with 60-80% survivors with colon tumour 26; 170-200 with osteosarcome and with Lewis' tumour.

The crotoxin complex was administered intramuscularly every four days. The result depended on the stage of the illness. The animals treated immediately after the implantation of the tumour responded much better than those treated when the illness was advanced.

## PHARMACOKINETICS

Pharmacokinetics studies were carried out using crotoxin B labelled with $I^{125}$ and in some cases the complex was doubly labelled with $H^3$ or $C^{14}$ in crotoxin A by reaction with acetic anhydride.

### 6.1 Absorption

Oral administration proved to be ineffective. After labelling crotoxin B with $I^{125}$ and forming the complex with crotoxin A, the complex was encapsulated in sphyngomyeline liposomes and administered. Under these conditions, measurable levels of crotoxin in plasma could be detected after oral administration, but the absorbed quantity varied considerably. Consequently, oral administration has been at present discarded.

After intravenous injection to mice and rabbits, plasmatic concentration falls rapidly, arriving at 2% of the original amount within approximately 30 minutes. About 30% is recovered in urine.

After intramuscular administration, a peak of plasmatic concentration is observed about 30 minutes after the injection. Within the hour, the plasmatic concentration is reduced to about 10% of the original amount and only traces thereof are detected in urine. Crotoxin B is mainly concentrated in the liver (HABERMANN, 1972, FRAENKEL-CONRAT et al., 1976), wherein it is degraded, passing to the aminoacids pool. The introduced label disappears almost completely between 6 and 8 hours after innoculation.

According to the results of HABERMANN et al (1972), crotoxin B or its complex with crotoxin A does not pass the hematoencephalic barrier as the amount of label measurable in cerebrum or spinal chord of intravenously innoculated is negligible. HABERMANN and RAUDE have demonstrated that injection of crotoxin in cerebral ventricles produces convulsions.

The above data suggests that crotoxin is distributed extensively, although it has a short average life and no persistent tissue concentrations appear.

Due to the rapid rate with which the organism catabolyses the complex, distribution studies were made administering high doses of labelled crotoxin complex ($I^{125}$ Crotoxin B) to albicans rodents. Two groups of animals were sacrificed 30 and 60 minutes after the intravenous administration of 220 $\mu$g of crotoxin complex to determine the concentration of the label in different organs and tissues. Concentrations are expressed in Femtomoles ($10^{-12}$ mol).

| TISSUE OR ORGAN | POST-INNOCULATION TIME | |
|---|---|---|
| | 30 Min | 60 Min |
| Spleen | 17500 | 2600 |
| Cerebrum and Spinal Medula | ND[a] | ND[a] |
| Heart | 3210 | 118 |
| Stomach | 216 | ND[a] |
| Lymphatic Ganglions | 230[b] | ND[a] |
| Mesenteric Fat | 280[b] | ND[a] |
| Liver | 2208000 | 1560200 |
| Hypophysis | ND[a] | ND[a] |
| Small Intestine | 366 | ND[a] |
| Large Intestine | 154 | ND[a] |
| Bone Marrow | 770[b] | ND[a] |
| Skeletal Muscle | 178000 | 71000 |
| Urine | 354000[c] | 2000[c] |
| Pancreas | 1620 | 124 |
| Lungs | 68000 | 780 |
| Kidney | 710000 | 266200 |
| Suprarrenal | 86 | ND[a] |
| Thyroid | ND[a] | ND[a] |
| Gallbladder | ND[a] | ND[a] |
| Total Recovered | 3516432 | 1903052 |
| Percentage of Total | 84.5% | 45.4% |

Note: The sacrified animals exhibited serious respiratory insufficiency and were kept alive with artificial respiration.

ND[a]: Non-detectable (<70)

$b_{DE}$: Average estimated data.

c: Appearance in urine in due to high dosage employed. As the molecular weight of the complex is 24000, it is filtered by the kidney.

6.2 Biotransformation

Biotransformation took place in the liver.

6.3 Excretion and final metabolites

The biotransformation products are aminoacids thus they enter the metabolic pool to be used by the organism.

Studies carried out on cell culture

The mechanism producing lysis in the tumoral cell can be explained as the result of enzymatic activity of crotoxin B; however, the point of greatest interest resides in the target selection mechanism (the malignant cell) by the crotoxin complex.

It is considered that the crotoxin complex acts as a circulating deposit of inert crotoxin B and manifests activity only on cells having defined physicochemical conditions, this resulting in the dissociation of the crotoxin complex and in the cell being exposed to the attack of crotoxin B. Under these conditions, a neoplastic cell would be a more efficient alternative "target" for capturing crotoxin B.

The therapeutic composition can be realised following commercial techniques, such as liquid formulations for instance in aqueous vehicles having the usual additives (ie, physiological solution), solution or emulsion eventually with other medicaments such as local anaesthetics, etc.

The objects and advantages of the present invention are illustrated in the following example, although reactive products and amounts detailed therein as well as conditions and other operative details are provided by way of example only, it being understood that said example does not limit the scope of the

invention.

EXAMPLE

I. Obtainment of the crude complex

500 mg of lyophilised material are suspended in 5 ml of 0.2 M sodium chloride solution, 1 mM disodium ethylenediamine tetraacetate, and 20 mM ammonium formate buffer pH 4.0. The solution is centrifuged at 10000 g for 20 minutes in a refrigerated Sorvall RC 2-B centrifuge at 4° C, the yellowish and slightly turbid supernatant is aspirated with a plastic syringe coupled to a Teflon tube constitute the starting material.

The remainder of the procedure is carried out in a cold chamber at 2 - 4° C.

Step 1:

The starting material is applied to a Sephadex G-75 column (Pharmacia Ltd. Uppsala, Sweden) measuring 85 x 2.5 cm (417 ml) adapted for upward flow and preequilibrated with 0.1 M ammonium formate buffer, pH 4.5 containing 0.1 mM disodium ethylenediamine tetraacetate. The elution is carried out with the same buffer solution, collecting fractions of 3.0 - 3.2 ml at a flow rate of 0.8 - 1.0 ml/cm$^2$/hour. The crude complex is eluted at a $K_{av}$ value ($K_{av}$ = $(V_e .V_o)/(V_o - V_t)$) of 0.44.

Fractions containing the crude complex are combined and concentrated by (a) lyophilization or (b) by ultrafiltration under nitrogen pressure in a chamber fitted with a Diaflo UM-10 membrane (Amicon Co., Danvers, Ma. USA) to a final volume of about 10 ml.

II. Separation of sub-units A and B from the crude complex

Step 2:

The sample obtained in Step 1 is adjusted to pH 3.5 with acetic acid and is applied to a CM-Sephadex C-50 column (Pharmacia Ltd., Uppsala, Sweden) having 8 x 2 cm and pre-equilibrated with 0.1 M ammonium formate buffer, pH 3.5. Elution starts with 250 ml 0.1 M ammonium formate buffer, pH 3.5 to elute sub-unit A which, under these conditions, is not retained by the exchanger. The elution is continued with 350 ml of a concave molarity gradient from 0.1 to 1.0 M ammonium formate, pH 3.5, and finally with 220 ml of a linear gradient from 1.0 to 3.0 M ammonium formate, pH 3.5. Five ml fractions were collected at a flow rate of 34 ml/cm$^2$/hour. The elution profile is followed by measuring the absorbance at 280 nm of eluate. The last peak of protein eluted under these conditions corresponds to sub-unit B.

III. Purification of Sub-units

Step 3: Purification of sub-unit A:

The fraction corresponding to the first peak to be eluted from Step 2 contains sub-unit A. This and other fractions containing sub-unit A were combined and dialyzed against 3 x 1000 ml of 10 mM potassium phosphate, pH 6.9. Alternatively, the combined fractions are concentrated by ultrafiltration under nitrogen pressure on a Diaflo UM-05 (Amicon Co., Danvers, Ma. USA) and equilibrated with 10 mM potassium phosphate, pH 6.9 buffer by chromatography of Sephadex G-25 (Pharmacia Ltd., Uppsala, Sweden).

The sample is applied to a DEAE-Sephadex A-50 column (10 x 1.5 cm) pre-equilibrated with 10 mM potassium phosphate pH 6.9 buffer. Sub-unit A is eluted by 200 ml of a linear gradient (100 ml of 10 mM potassium phosphate, pH 6.9 + 100 ml 0.1 mM sodium phosphate, pH 6.9).

Fractions containing purified sub-unit A are extensively dialyzed against 3 x 100 ml of 0.15 M NaCl and concentrated by ultrafiltration as previously mentioned.

The recovery of sub-unit A is 37.5 mg of protein. As obtained under the above conditions, sub-unit A exhibits only one precipitation line by immuno-electrophoresis on agar gel.

Step 4: Purification of Sub-unit B

Fractions containing sub-unit B (eluted last in Step 2) are combined and the sample is lyophilized or dialyzed against 2 x 1000 ml of 0.1 M ammonium formate buffer, pH 3.5 and chromatographed on a CM-Sephadex C-50 column pre-equilibrated with the same buffer as described in Step 2 with the exception that, once concluded the concave gradient of ammonium formate up to 0.1 M, the elution is continued with ammonium formate buffer (0.2 M, pH 3.5) so as to concentrate the active fraction.

The fractions containing purified sub-unit B are extensively dialyzed against 6 x 1000 ml of 0.15 M NaCl.

The recovery of sub-unit B is 75 mg of protein. As obtained under the above conditions and subjected to polyacrylamide gel electrophoresis with sodium dodecylsulphate, it exhibits two bands with mobilities corresponding to molecular weights of 14500 (monomer) and 29000 (dimer).

Sterilization of purified sub-units:

The preparations of sub-units A and B purified in a 0.15 M NaCl solution are sterilized by filtration through Millipore membranes in an ultraviolet chamber. Reconstitution of the complex starting from the purified sub units:

Sub-units A and B spontaneously form a complex having a dissociation constant in the order of $10^{-10}$ M and same is completed within the first minute after mixture.

Considering the results obtained in the use of the crotoxin complex for treating advanced cancer, the following conclusions are arrived at:

From the viewpoint of basic investigation

Concerning the cytotoxic action, it has been demonstrated that preferential cytotoxicity of crotoxin occurs with respect to tumor cells.
The final metabolytes of the crotoxin complex are aminoacids.
In the use of the crotoxin complex, no significant immune response has been detected either in animals or human beings.
Broad spectrum of antitumor activity.
No serious toxicity effects were observed, either acute, sub-acute or cronically accumulative.
No psycho-organic dependency has been detected.
The complex does not interact with other treatments, according to the above clinical experience with patients undergoing several treatments.
There are no counterindications. Experimental animals submitted to intravenous injection exhibited acute tubular nephropathy which, in some cases, caused death.
According to the pharmacokinetics, as the crotoxin complex is of low molecular weight proteins (P.M.), it might be absorbed by the renal tubules causing kidney damage.
To date, the following administration routes have been tried and found to be effective and safe: intramuscular, intratumoral, peri-tumoral, intracavitary, through fistular sections, intradermically, intrachirurgical and intramucosa.
After metabolization, there is no accumulation in any organ.
No effects attributable to tumor lysis have been observed, such as hyperuricemia, hypercalcamia, etcetera.
Administration may be daily and does not require a special technique or application procedure.
Administration may be carried out in ambulatory conditions.
Crotoxin is not bound to plasmatic proteins for transport.
No hemolysis or alterations in blood coagulation have been observed.
No electrocardiographic alterations or hemodynamic failures have been registered.

**Claims**
**Claims for the following Contracting States : BE, CH, DE, FR, GB, IT, LI, NL, SE**

1. The use of a substantially pure crotoxin complex of crotoxin A and B sub-units for the manufacture of a therapeutic composition for the treatment of malignant tumours.

2. The use as claimed in claim 1, wherein the complex is produced by recovering the crotoxin sub-units A and B separately from Crotalus venom, purifying the sub-units and then combining the purified sub-units to form the crotoxin complex.

3. The use as claimed in claim 1 or 2, in combination with cobrame or melittin.

4. A procedure for manufacturing a therapeutic composition containing a crotoxin complex of crotoxin A and B sub-units for the treatment of malignant tumours, said procedure comprising the steps of separating by chromatography the crotoxin complex from crude Crotalus venom; concentrating reunited fractions formed by eluting the absorbed crotoxin complex; dispersing in aqueous medium an insoluble product precipitated in said concentrating step; fractionating said sub-units A and B by chromatography of said dispersion in an adsorption column and eluting sub-units A and B with a buffer solution; purifying the separated eluted fractions containing said sub-units A and B; and eventually mixing said units to form the crotoxin complex.

5. A process for preparing a therapeutic composition for the treatment of malignant tumours, which process comprising the step of mixing a therapeutically effective amount of a substantially pure crotoxin complex of crotoxin A and B sub-units with a pharmacologically acceptable vehicle.

6. The use of purified crotoxin B for the manufacture of a therapeutic composition for the treatment of malignant tumours.

**Claims for the following Contracting States : AT, ES, GR**

1. A process for preparing a therapeutic composition for the treatment of malignant tumours, said process comprising the step of mixing a therapeutically effective amount of a substantially pure crotoxin complex of crotoxin A and B sub-units with a pharmacologically acceptable vehicle.

2. A process according to Claim 1, further including the steps of recovering sub-units A and B separately from Crotalus venom, purifying the sub-units, and then combining the purified sub-units to form the crotoxin complex.

3. A process according to Claim 1 or 2, further comprising the step of including melittin or cobrame in the composition.

4. A process according to Claim 2, comprising the steps of separating by chromatography the crotoxin complex from crude Crotalus venom; concentrating reunited fractions formed by eluting the absorbed crotoxin complex; dispersing in aqueous medium an insoluble product precipitated in said concentration stage; fractioning said sub-units A and B by chromatography of said dispersion in an adsorption column and eluting sub-units A and B with a buffer solution; purifying the separated eluted fractions containing said sub-units A and B; and eventually mixing said units to form a crotoxin complex of said sub-units A and B.

5. A process for preparing a therapeutic composition for the treatment of malignant tumours, said process comprising the step of mixing a therapeutic amount of purified crotoxin B with a pharmacologically acceptable vehicle.

**Patentansprüche**
**Patentansprüche für folgende Vertragsstaaten : BE, CH, DE, FR, GB, IT, LI, NL, SE**

1. Verwendung eines im wesentlichen reinen Crotoxin-Komplexes aus Crotoxin A- und B-Untereinheiten zur Herstellung einer therapeutischen Zusammensetzung für die Behandlung von malignen Tumoren.

2. Verwendung nach Anspruch 1, wobei der Komplex durch getrennte Gewinnung der Crotoxin-Untereinheiten A und B aus Crotalus-Gift, Reinigen der Untereinheiten und dann Verbinden der gereinigten Untereinheiten zur Bildung des Crotoxin-Komplexes hergestellt wird.

3. Verwendung nach Anspruch 1 oder 2 in Verbindung mit Cobrame oder Melittin.

4. Verfahren zum Herstellen einer therapeutischen Zusammensetzung, enthaltend einen Crotoxin-Komplex aus Crotoxin A- und B-Untereinheiten für die Behandlung maligner Tumoren, wobei das Verfahren die Schritte umfaßt: chromatographische Abtrennung des Crotoxin-Komplexes aus rohem Crotalus-Gift, Konzentrieren der wiedervereinigten Fraktionen, die durch Eluieren des absorbierten Crotoxin-Komplexes gebildet werden, Dispergieren eines in dem Konzentrationsschritt präzipitierten unlöslichen Produktes in einem wäßrigen Medium, Fraktionieren der Untereinheiten A und B durch Chromatographie der Dispersion auf einer Adsorptionssäule und Eluieren der Untereinheiten A und B mit einer Pufferlösung, Reinigen der getrennten eluierten Fraktionen, die die Untereinheiten A und B enthalten, und schließlich Mischen der Einheiten zur Bildung des Crotoxin-Komplexes.

**5.** Verfahren zum Herstellen einer therapeutischen Zusammensetzung für die Behandlung von malignen Tumoren, wobei das Verfahren den Schritt des Mischens einer therapeutisch wirksamen Menge eines im wesentlichen reinen Crotoxin-Komplexes aus Crotoxin A- und B-Untereinheiten mit einem pharmakologisch verträglichen Träger umfaßt.

**6.** Verwendung von gereinigten Crotoxin B für die Herstellung einer therapeutischen Zusammensetzung für die Behandlung von malignen Tumoren.

**Patentansprüche für folgende Vertragsstaaten : AT, ES, GR**

**1.** Verfahren zum Herstellen einer therapeutischen Zusammensetzung für die Behandlung von malignen Tumoren, wobei das Verfahren den Schritt des Mischens einer therapeutisch wirksamen Menge eines im wesentlichen reinen Crotoxin-Komplexes aus Crotoxin A- und B-Untereinheiten mit einem pharmakologisch verträglichen Träger umfaßt.

**2.** Verfahren nach Anspruch 1, das weiterhin die Schritte umfaßt: getrenntes Gewinnen der Untereinheiten A und B aus Crotalus-Gift, Reinigen der Untereinheiten und dann Verbinden dar gereinigten Untereinheiten zur Bildung des Crotoxin-Komplexes.

**3.** Verfahren nach Anspruch 1 oder 2, das weiter den Schritt des Einbeziehens von Melittin oder Cobrame in die Zusammensetzung umfaßt.

**4.** Verfahren nach Anspruch 2, umfassend die Schritte: chromatographisches Abtrennen des Crotoxin-Komplexes aus rohem Crotalus-Gift, Konzentrieren der wiedervereinigten Fraktionen, die durch Eluieren des absorbierten Crotoxin-Komplexes gebildet werden, Dispergieren eines in dem Konzentrationsschritt präzipitierten unlöslichen Produktes in einem wäßrigen Medium, Fraktionieren der Untereinheiten A und B durch Chromatographie der Dispersion auf einer Adsorptionssäule und Eluieren der Untereinheiten A und B mit einer Pufferlösung, Reinigen der getrennten eluierten Fraktionen, die die Untereinheiten A und B enthalten, und schließlich Mischen der Einheiten zur Bildung des Crotoxin-Komplexes aus den Untereinheiten A und B.

**5.** Verfahren zum Herstellen einer therapeutischen Zusammensetzung für die Behandlung von malignen Tumoren, wobei das Verfahren den Schritt des Mischens einer therapeutischen Menge von gereinigtem Crotoxin B mit einem pharmakologisch verträglichem Träger umfaßt.

**Revendications**
**Revendications pour les Etats contractants suivants : BE, CH, DE, FR, GB, IT, LI, NL, SE**

**1.** Utilisation d'un complexe de crotoxine sensiblement pur de sous-unités de crotoxine A et B en vue de la préparation d'une composition thérapeutique destinées au traitement de tumeurs malignes.

**2.** Utilisation suivant la revendication 1, caractérisée en ce que le complexe est produit en récupérant les sous-unités A et B de la crotoxine séparément du venin de crotale, en purifiant les sous-unités et en combinant les sous-unités purifiées pour former le complexe de crotoxine.

**3.** Utilisation suivant la revendication 1 ou 2, en combinaison avec du cobrame ou de la mélittine.

**4.** Procédé de préparation d'une composition thérapeutique contenant le complexe de crotoxine de sous-unités de crotoxine A et B en vue du traitement de tumeurs malignes, caractérisé en ce qu'il comprend les étapes de séparation par chromatographie du complexe de crotoxine à partir du venin de crotale brut, de concentration des fractions réunies formées par l'élution du complexe de crotoxine absorbé, de dispersion en milieu aqueux d'un produit insoluble précipité au cours de ladite étape de concentration, de fractionnement desdites sous-unités A et B par chromatographie de ladite dispersion dans une

colonne d'adsorption et d'élution des sous-unités A et B avec une solution tampon, de purification des fractions éluées séparées contenant lesdites sous-unités A et B et déventuel mélange desdites unités pour former le complexe de crotoxine.

**5.** Procédé de préparation d'une composition thérapeutique destinée au traitement de tumeurs malignes, caractérisé en ce qu'il comprend l'étape de mélange d'une proportion thérapeutiquement efficace d'un complexe de crotoxine sensiblement pur de sous-unités de crotoxine A et B avec un véhicule pharmacologiquement acceptable.

**6.** Utilisation de la crotoxine B purifiée pour la préparation d'une composition thérapeutique destinée au traitement de tumeurs malignes.

**Revendications pour les Etats contractants suivants : AT, ES, GR**

**1.** Procédé de préparation d'une composition thérapeutique destinée au traitement de tumeurs malignes, caractérisé en ce qu'il comprend l'étape de mélange d'une proportion thérapeutiquement efficace d'un complexe de crotoxine sensiblement pur de sous-unités de crotoxine A et B avec un véhicule pharmacologiquement acceptable.

**2.** Procédé suivant la revendication 1, caractérisé en ce qu'il comprend aussi les étapes de récupération des sous-unités A et B séparément du venin de crotale, de purification des sous-unités et de combinaison subséquente des sous-unités purifiées pour former le complexe de crotoxine.

**3.** Procédé suivant la revendication 1 ou 2, caractérisé en ce qu il comprend aussi l'étape d'incorporation de mélittine ou de cobrame à la composition.

**4.** Procédé suivant la revendication 2, caractérisé en ce qu'il comprend les étapes de séparation par chromatographie du complexe de crotoxine du venin de crotale brut, de concentration des fractions réunies formées par l'élution du complexe de crotoxine absorbé, de dispersion dans un milieu aqueux d'un produit insoluble précipité au cours de l'étape de concentration précitée, de fractionnement desdites sous-unités A et B par chromatographie de ladite dispersion dans une colonne d'adsorption et d'élution des sous-unités A et B avec une solution tampon, de purification des fractions éluées séparées contenant lesdites sous-unités A et B et d'éventuel mélange desdites unités pour former un complexe de crotoxine desdites sous-unités A et B.

**5.** Procédé de préparation d'une composition thérapeutique destinée au traitement de tumeurs malignes, caractérisé en ce qu'il comprend l'étape de mélange d'une proportion thérapeutique de crotoxine B purifiée à un véhicule pharmacologiquement acceptable.